(19) **Europäisches Patentamt / European Patent Office / Office européen des brevets**

(11) **EP 4 659 682 A1**

(12) **EUROPEAN PATENT APPLICATION**
published in accordance with Art. 153(4) EPC

(43) Date of publication:
**10.12.2025 Bulletin 2025/50**

(21) Application number: **23919906.0**

(22) Date of filing: **29.11.2023**

(51) International Patent Classification (IPC):
**A61B 6/42** $^{(2024.01)}$     **A61B 6/00** $^{(2024.01)}$
**H05G 1/44** $^{(2006.01)}$

(52) Cooperative Patent Classification (CPC):
**A61B 6/482; A61B 6/00; A61B 6/42; A61B 6/542; H05G 1/44;** A61B 6/4035; A61B 6/4241; A61B 6/4258

(86) International application number:
**PCT/JP2023/042779**

(87) International publication number:
**WO 2024/161771 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.02.2023 JP 2023014741**

(71) Applicant: **FUJIFILM Corporation**
**Tokyo 106-8620 (JP)**

(72) Inventor: **TAKAHASHI, Tomoyuki**
**Ashigarakami-gun, Kanagawa 258-8538 (JP)**

(74) Representative: **Dehns Germany Partnerschaft mbB**
**Theresienstraße 6-8**
**80333 München (DE)**

(54) **RADIOGRAPHIC IMAGING CONTROL DEVICE, METHOD, AND PROGRAM**

(57)     A radiography control device that controls radiography of an imaging target by irradiating the imaging target with radiation emitted from a radiation source is provided. The radiography control device includes a layer structure detector configured by stacking a plurality of radiation detectors each having a plurality of dose detection pixels for detecting a dose during radiography, and at least one processor. The processor acquires an imaging purpose, determines at least one radiation detector to be used for dose control of radiation during radiography among the plurality of radiation detectors according to the imaging purpose, and performs the dose control according to a dose detected by a dose detection pixel of the determined radiation detector.

**FIG. 5**

RADIOGRAPHY CONTROL DEVICE — 10

DOSE CONTROLLER — 21

DEVICE CONTROLLER — 22

SUBTRACTION UNIT — 23

DISPLAY CONTROLLER — 24

EP 4 659 682 A1

## Description

BACKGROUND OF THE INVENTION

1. Field of the Invention

The present disclosure relates to a radiography control device, a radiography control method, and a radiography control program.

2. Description of the Related Art

In the related art, a radiation image is acquired and used for diagnosis by detecting radiation transmitted through a subject with a radiation detector such as a flat panel detector (FPD). In such a system, a radiography system comprising an auto exposure control (AEC) mechanism that detects a radiation dose reaching a radiation detector and stops the irradiation of the radiation in a case in which the radiation dose reaches a predetermined value has been proposed (for example, see JP2013-233420A). By using such a system, the radiation dose reaching the radiation detector can be controlled to a constant level, and a radiation image suitable for diagnosis can always be obtained.

On the other hand, energy subtraction processing using two radiation images obtained by irradiating a subject with two types of radiation having different energy distributions by using the fact that an attenuation amount of the transmitted radiation differs depending on the substance constituting the subject has been known. As a radiation detector for performing such energy subtraction processing, a layer structure detector has been proposed. The layer structure detector is configured by, for example, stacking two radiation detectors in a layer shape. It is possible to perform energy subtraction imaging with one irradiation of radiation by using such a layer structure detector. In addition, it is also possible to perform simple imaging similar to a case in which only one radiation detector is used, by acquiring the radiation image using only a radiation detector on the side close to the radiation source.

SUMMARY OF THE INVENTION

However, in a case in which the layer structure detector is used, the radiation dose irradiated onto the radiation detector on the side far from the radiation source is smaller than the radiation dose applied to the radiation detector on the side close to the radiation source. Therefore, in a case in which the dose is not appropriately set in a case in which the energy subtraction processing is performed, the radiation dose with which the radiation detector on the side far from the radiation source is irradiated is too small, so that the signal-to-noise ratio (S/N) is decreased. As a result, the image quality of the radiation image acquired by the energy subtraction processing is decreased.

The present disclosure has been made in view of the above circumstances, and an object of the present disclosure is to enable acquisition of a high-quality radiation image in a case in which radiography is performed using a layer structure detector.

According to the present disclosure, there is provided a radiography control device that controls radiography of an imaging target by irradiating the imaging target with radiation emitted from a radiation source, the radiography control device comprising: a layer structure detector configured by stacking a plurality of radiation detectors each having a plurality of dose detection pixels for detecting a dose during the radiography; and at least one processor, in which the processor is configured to: acquire an imaging purpose; determine at least one radiation detector to be used for dose control of the radiation during the radiography among the plurality of radiation detectors according to the imaging purpose; and perform the dose control according to the dose detected by the dose detection pixel of the determined radiation detector.

In the radiography control device according to the present disclosure, the processor may be configured to determine a radiation detector closest to the radiation source among the plurality of radiation detectors as the radiation detector for performing the dose control, in a case in which the imaging purpose is simple imaging.

In addition, in the radiography control device according to the present disclosure, the processor may be configured to determine a radiation detector other than a radiation detector closest to the radiation source among the plurality of radiation detectors as the radiation detector for performing the dose control, in a case in which the imaging purpose is energy subtraction imaging.

In addition, in the radiography control device according to the present disclosure, the processor may be configured to determine all of the plurality of radiation detectors as the radiation detector for performing the dose control, in a case in which the imaging purpose is energy subtraction imaging.

In the radiography control device according to the present disclosure, the processor may be configured to: determine a target dose in a case in which the radiography is performed according to the imaging purpose; and stop driving of the radiation source in a case in which the dose detected by the dose detection pixel of the determined radiation detector reaches the target dose.

In addition, in the radiography control device according to the present disclosure, the processor may be configured to: specify a region of interest in the imaging target according to the imaging purpose; and perform the dose control according to the dose detected by the dose detection pixel at least at a position corresponding to the region of interest in the radiation detector determined as the radiation detector that controls the dose of the radiation.

According to the present disclosure, there is provided a radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method comprising: acquiring an imaging purpose; determining at least one radiation detector to be used for dose control of the radiation during the radiography among the plurality of radiation detectors according to the imaging purpose; and performing the dose control according to the dose detected by the dose detection pixel of the determined radiation detector.

According to the present disclosure, there is provided a radiography control program causing a computer to execute a radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method including: a procedure of acquiring an imaging purpose; a procedure of determining at least one radiation detector to be used for dose control of the radiation during the radiography among the plurality of radiation detectors according to the imaging purpose; and a procedure of performing the dose control according to the dose detected by the dose detection pixel of the determined radiation detector.

According to the present disclosure, it is possible to acquire a high-quality radiation image in a case in which radiography is performed using a layer structure detector.

BRIEF DESCRIPTION OF THE DRAWINGS

Fig. 1 is a schematic block diagram showing a configuration of a radiography system to which a radiography control device according to an embodiment of the present disclosure is applied.
Fig. 2 is a diagram showing a schematic configuration of a radiation detector.
Fig. 3 is a diagram for describing a distribution of dose detection pixels.
Fig. 4 is a diagram showing a schematic configuration of a radiography control device according to the present embodiment.
Fig. 5 is a diagram showing a functional configuration of the radiography control device according to the present embodiment.
Fig. 6 is a diagram for describing a region of interest of a chest.

Fig. 7 is a flowchart showing processing performed in the present embodiment.
Fig. 8 is a flowchart showing processing that is performed in another embodiment.

DESCRIPTION OF THE PREFERRED EMBODIMENTS

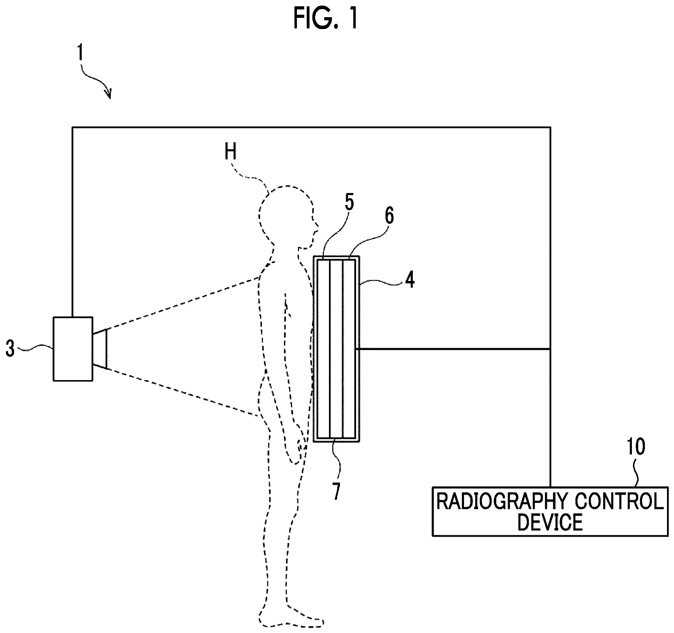

In the following, an embodiment of the present disclosure will be explained with reference to the drawings. Fig. 1 is a schematic block diagram showing a configuration of a radiography system to which a radiography control device according to the present embodiment of the present disclosure is applied. As shown in Fig. 1, the radiography system according to the present embodiment comprises an imaging apparatus 1 and a radiography control device 10 according to the present embodiment.

The imaging apparatus 1 comprises a radiation source 3 and a layer structure detector 4. The layer structure detector 4 is configured by stacking a first radiation detector 5, a radiation energy conversion filter 7 consisting of a copper plate or the like, and a second radiation detector 6 in order from a side close to the radiation source 3.

By using such a layer structure detector 4, in the imaging apparatus 1, it is possible to perform energy subtraction by a so-called one-shot method of irradiating the first radiation detector 5 and the second radiation detector 6 with radiation, such as X-rays, which is emitted from the radiation source 3 and transmitted through a subject H, which is an imaging target, with different energies. In addition, in a case in which only the first radiation detector 5 of the layer structure detector 4 is used, it is possible to acquire a radiation image by performing simple imaging of the subject H.

Here, the energy subtraction is processing of generating an image in which different tissues (for example, a soft part and a bone part) in the subject are extracted by using two radiation images obtained by irradiating the subject with two types of radiation having different energy distributions by using the fact that an attenuation amount of the transmitted radiation differs depending on the substance constituting the subject.

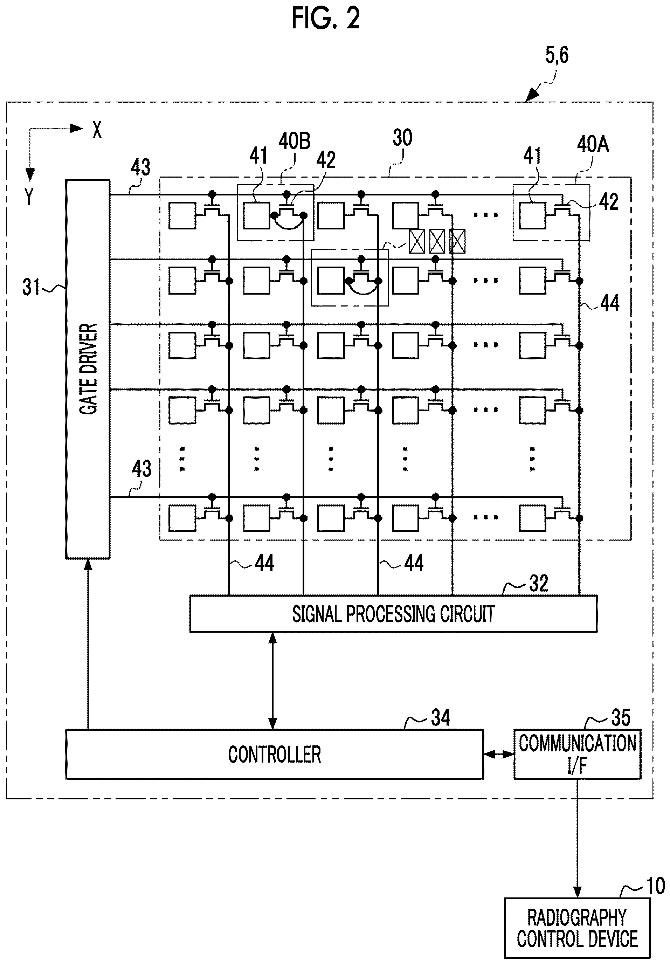

Fig. 2 is a diagram showing a schematic configuration of the first and second radiation detectors. In a case in which it is not necessary to distinguish between the first radiation detector and the second radiation detector, the first radiation detector and the second radiation detector may be simply referred to as a radiation detector. As shown in Fig. 2, the radiation detectors 5 and 6 have a pixel region 30, a gate driver 31, a signal processing circuit 32, a controller 34, and a communication interface (I/F) 35.

The pixel region 30 has a plurality of normal pixels 40A arranged in a matrix along an X direction and a Y direction, which are orthogonal to each other. The normal pixel 40A is a pixel for image generation for detecting radiation and generating a radiation image. In addition, a plurality of dose detection pixels 40B are provided in the pixel region 30 in addition to the normal pixel 40A. The dose detection pixel 40B is a pixel for detecting a dose of the radiation emitted to the radiation detectors 5 and 6.

The normal pixel 40A includes a photoelectric conversion unit 41 that performs photoelectric conversion on the visible light converted by the scintillator to generate and accumulate the electric charges and a TFT 42 as a switching element. The photoelectric conversion unit 41 includes, for example, a p-intrinsic-n (PIN) semiconductor layer, an upper electrode that is disposed above the semiconductor layer, and a lower electrode that is disposed below the semiconductor layer. A bias voltage is applied to the upper electrode. The lower electrode is connected to a thin film transistor (TFT) 42.

The dose detection pixel 40B has the photoelectric conversion unit 41 and the TFT 42, similar to the normal pixel 40A. However, in the dose detection pixel 40B, a source electrode and a drain electrode of the TFT 42 are short-circuited. The dose detection pixel 40B is a pixel used to detect a reaching dose of radiation transmitted through the subject H and incident on imaging surfaces of the radiation detectors 5 and 6, and functions as an AEC sensor for stopping the irradiation with radiation as will be described later.

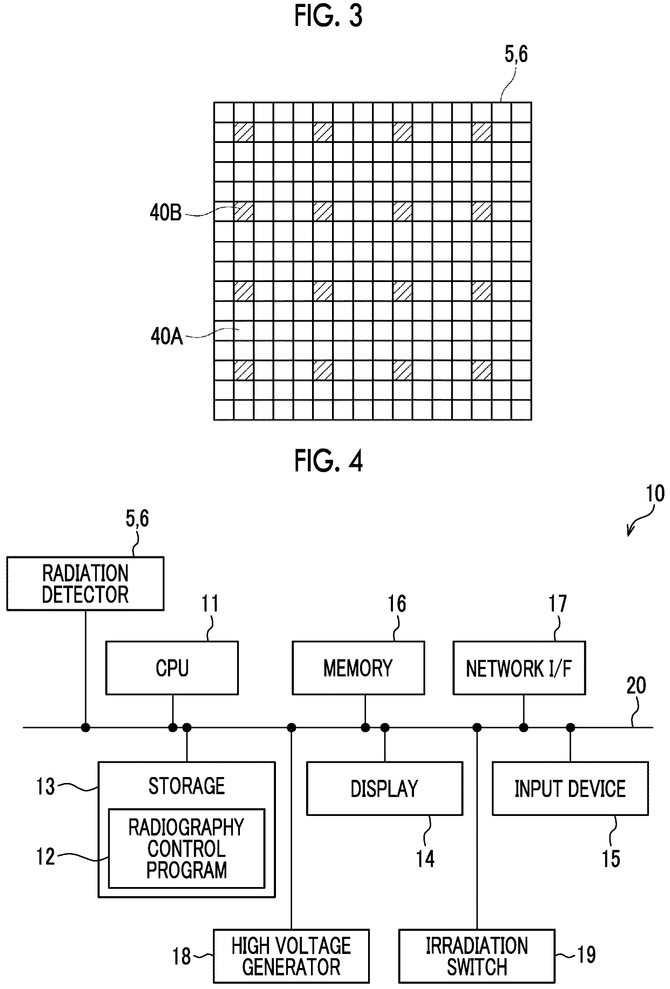

The dose detection pixel 40B occupies approximately a few percent of the pixels included in the imaging surfaces of the radiation detectors 5 and 6. It is preferable that the dose detection pixels 40B are provided to be evenly scattered in the imaging surface without being locally biased in the imaging surface. For example, as shown in Fig. 3, it is preferable that the dose detection pixels 40B are provided to be evenly distributed in the imaging surface at intervals of several pixels. The positions of the dose detection pixels 40B are known at the time of manufacturing the radiation detectors 5 and 6, and are preferably stored in advance in a non-volatile memory, which will be described later. The dose detection pixels 40B may be locally concentrated and disposed, and the disposition of the dose detection pixels 40B can be changed as appropriate. In the following, in a case in which it is not necessary to distinguish between the normal pixel 40A and the dose detection pixel 40B, these are simply referred to as a pixel 40.

In the example shown in Fig. 2, the dose detection pixels 40B are disposed at intervals of several pixels vertically and horizontally instead of the normal pixels at the positions of the normal pixels for image detection of the radiation detectors 5 and 6. However, the present disclosure is not limited to this, and the dose detection pixels 40B may be disposed in gaps between the normal pixels 40A. In this case, since it is not necessary to use the positions of the normal pixels as the dose detection pixels 40B, the pixel density can be increased accordingly.

The pixel region 30 has a plurality of scanning lines 43 that extend in the X direction and a plurality of signal lines 44 that extend in the Y direction. The scanning lines 43 and the signal lines 44 are wired in a lattice form. Each pixel 40 is connected to an intersection portion of the scanning line 43 and the signal line 44. Specifically, in the pixel 40, the gate electrode of the TFT 42 is connected to the scanning line 43, and the source electrode of the TFT 42 is connected to the signal line 44. In addition, the drain electrode of the TFT 42 is connected to the photoelectric conversion unit 41.

Each scanning line 43 is commonly connected to the pixels 40 corresponding to one pixel row. Each signal line 44 is commonly connected to the pixels 40 corresponding to one pixel column. Each scanning line 43 is connected to the gate driver 31. Each signal line 44 is connected to the signal processing circuit 32.

The gate driver 31 supplies a gate pulse as a scanning signal to each scanning line 43 in order. The gate pulse supplied to the scanning line 43 is applied to the gate electrode of the TFT 42 included in the pixel 40 connected to the scanning line 43.

The electric charges accumulated in the photoelectric conversion unit 41 of the normal pixel 40A are output to the signal line 44 in a case in which the TFT 42 is in an on state. Since the source electrode and the drain electrode of the TFT 42 in the dose detection pixel 40B are short-circuited, the electric charge generated in the photoelectric conversion unit 41 of the dose detection pixel 40B is output to the signal line 44 regardless of the switching state of the TFT 42.

The signal processing circuit 32 has an integrator as a charge amplifier, a correlated double sampling (CDS) circuit, and an analog/digital (A/D) converter. The signal processing circuit 32 integrates the electric charge input from each pixel 40 via the signal line 44 using an integrator, and then performs the correlated double sampling using the CDS circuit. Then, the signal processing circuit 32 converts the pixel signal from which the reset noise component is removed by the correlated double sampling into a digital signal using the A/D converter.

The signal processing circuit 32 generates image data of the radiation image based on the pixel signals read out from each normal pixel 40A of the pixel region 30. On the other hand, the electric charge generated in the dose detection pixel 40B always flows into the integrator on the signal line to which the dose detection pixel 40B in the signal processing circuit 32 is connected. The signal processing circuit 32 generates dose data for performing dose control, as described below, based on the pixel signals read out from the dose detection pixel 40B.

The controller 34 is configured by a microcomputer, and comprises a central processing unit (CPU), a memory, and a storage device. The controller 34 performs control for radiography by executing a program stored in the memory by the CPU. The controller 34 controls each part of the gate driver 31, the signal processing circuit 32, and the communication I/F 35.

In a case in which the imaging is started, the controller 34 controls the gate driver 31 and the signal processing circuit 32 to perform a reset operation of the electric charges accumulated in the normal pixel 40A. Specifically, the controller 34 outputs the accumulated electric charge of each normal pixel 40A to the signal line 44 by supplying the gate pulse to each scanning line 43 from the gate driver 31, and discards the electric charge in the signal processing circuit 32. After the reset operation ends, the controller 34 sets all the TFTs 42 to the off state to set the normal pixel 40A to an electric charge accumulation state.

The controller 34 sets the normal pixel 40A to a charge accumulation state, and after the irradiation of the radiation is stopped as will be described below, the controller 34 controls the gate driver 31 to read out the pixel signal from the normal pixel 40A to the signal processing circuit 32, thereby generating image data of the radiation image. In addition, in a case in which the imaging is started, the controller 34 controls the signal processing circuit 32 and generates the dose data from the electric charges generated in the dose detection pixels 40B. The controller 34 outputs the radiation image and the dose data to the radiography control device 10 via the communication I/F 35.

In the radiography control device 10 according to the present embodiment, the first and second radiation detectors 5 and 6 included in the above-described layer structure detector 4 are switched for use according to the imaging purpose. That is, in a case in which the imaging purpose is simple imaging for acquiring one radiation image of the subject H, only the first radiation detector 5 on a side close to the radiation source 3 is used. On the other hand, in a case in which the imaging purpose is energy subtraction imaging, both the first radiation detector 5 and the second radiation detector 6 are used.

Next, the radiography control device according to the present embodiment will be described. First, a hardware configuration of the radiography control device according to the present embodiment will be described with reference to Fig. 4. As shown in Fig. 4, the radiography control device 10 is a computer, such as a workstation, a server computer, and a personal computer, and comprises a central processing unit (CPU) 11, a nonvolatile storage 13, and a memory 16 as a temporary storage area. In addition, the radiography control device 10 comprises a display 14, such as a liquid crystal display, an input device 15, such as a keyboard and a mouse, and a network interface (I/F) 17 connected to a network (not shown). In addition, the radiography control device 10 comprises a high voltage generator 18 and an irradiation switch 19 connected to the radiation source 3. The CPU 11, the storage 13, the display 14, the input device 15, the memory 16, the network I/F 17, the high voltage generator 18, and the irradiation switch 19 are connected to a bus 20. In addition, the radiation detectors 5 and 6 are also connected to the bus 20. It should be noted that the CPU 11 is an example of a processor according to the present disclosure.

The storage 13 is realized by a hard disk drive (HDD), a solid state drive (SSD), a flash memory, and the like. A radiography control program 12 installed in the radiography control device 10 is stored in the storage 13 as a storage medium. The CPU 11 reads out the radiography control program 12 from the storage 13, loads the radiography control program 12 in the memory 16, and executes the loaded radiography control program 12.

The high voltage generator 18 boosts an input voltage using a transformer to generate a high tube voltage, and supplies the high tube voltage to the radiation source 3 through a high voltage cable.

The irradiation switch 19 is, for example, a two-stage push switch operated by an operator such as a radiologist, and pressing it to the first stage generates a warm-up start signal for starting warm-up of the radiation source 3 and pressing it to second stage generates an irradiation start signal for starting irradiation of the radiation source 3.

The radiography control program 12 is stored in a storage device of the server computer connected to the network or in a network storage in a state of being accessible from the outside, and is downloaded and installed in the computer that configures the radiography control device 10 in response to the request. Alternatively, the radiography control program 12 is distributed in a state of being recorded on a recording medium, such as a digital versatile disc (DVD) or a compact disc read only memory (CD-ROM), and is installed in the computer that configures the radiography control device 10 from the recording medium.

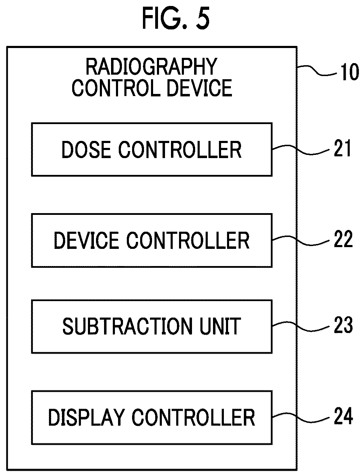

Next, a functional configuration of the radiography control device according to the present embodiment will be described. Fig. 5 is a diagram showing the functional configuration of the radiography control device according to the present embodiment. As shown in Fig. 5, the radiography control device 10 comprises a dose controller 21, a device controller 22, a subtraction unit 23, and a display controller 24. Then, the CPU 11 executes the radiography control program 12 to function as the dose controller 21, the device controller 22, the subtraction unit 23, and the display controller 24.

The dose controller 21 controls radiography by irradiating the subject H with radiation. Specifically, the radiation source 3 is driven by controlling a tube voltage that determines an energy spectrum of radiation emitted from the radiation source 3, a tube current that determines an irradiation amount per unit time, the start, stop, or end of irradiation of the radiation source 3, and an irradiation time of the radiation. That is, the dose controller 21 starts the supply of power from the high voltage generator 18 to the radiation source 3 in a case in which the irradiation start signal is received from the irradiation switch 19, stops the supply of power from the high voltage generator 18 to the radiation source 3 in a case in which the emitted dose reaches the target dose, and stops the irradiation of the radiation by the radiation source 3.

The storage 13 stores several types of imaging conditions, such as a tube voltage and a tube current, in advance according to the imaging part or the like. The imaging conditions are manually set by the operator through the input device 15. The dose controller 21 emits radiation based on the tube voltage and the tube current irradiation time product of the set imaging conditions. On the other hand, as will be described later, in a case in which it is detected that the irradiation dose based on the dose data has reached a necessary and sufficient dose, the dose controller 21 functions to stop the irradiation of radiation even in a case in which the dose is equal to or less than the tube current irradiation time product (irradiation time) which is intended to be used for irradiation based on the imaging conditions. In order to prevent a situation where the irradiation of the radiation ends before the target dose is reached and the irradiation stop signal of radiation is received, leading to a dose shortage, the maximum value of the tube current irradiation time product (or the irradiation time) is set as the imaging condition of the radiation source 3. It is preferable that the set tube current irradiation time product is set to a value corresponding to the imaging part.

On the other hand, the dose controller 21 acquires the imaging purpose for dose control. Examples of the imaging purpose include simple imaging of acquiring one radiation image of the subject H and energy subtraction imaging. The imaging purpose is set by the operator through the input device 15. In a case in which the imaging purpose is simple imaging, only the first radiation detector 5 on the side close to the radiation source 3 in the layer structure detector 4 is used to acquire the radiation image. On the other hand, in a case in which the imaging purpose is energy subtraction imaging, both the first radiation detector 5 and the second radiation detector 6 are used, and the first radiation detector 5 acquires a first radiation image G1 and the second radiation detector 6 acquires a second radiation image G2.

The dose controller 21 determines a radiation detector to be used for dose control according to the imaging purpose. In a case in which the imaging purpose is simple imaging, the first radiation detector 5 is determined as the radiation detector used for dose control. In a case in which the imaging purpose is energy subtraction imaging, the dose controller 21 determines the second radiation detector 6 as the radiation detector to be used for dose control such that the second radiation detector 6 on the side far from the radiation source 3 is irradiated with a sufficient amount of radiation.

A target dose in a case of performing the radiography may be determined according to the imaging purpose, and the dose control may be performed such that the subject H is irradiated with the radiation of the target dose. For example, in a case in which the imaging part is the chest in a case in which the imaging purpose is simple imaging, the lung field is the region of interest. For this reason, the target dose is determined such that the lung field in the radiation image acquired by the simple imaging has a desired image quality. Accordingly, a radiation image in which the lung field has a high image quality is generated.

The target dose in this case may be set as follows. That is, chest phantoms having various thicknesses, which are formed of acrylic or the like having the same radiation attenuation coefficient as that of the human body, are imaged while changing the dose. Then, in the radiation image acquired by the first radiation detector 5, in a case in which the lung field has a desired image quality, the average reaching dose in the lung field region is set to the target dose and is stored in the storage 13. In a case in which the imaging is actually performed, the target dose stored in the storage 13 and corresponding to the imaging purpose may be read out from the storage 13 and used.

In addition, in a case in which the imaging part is the chest in a case in which the imaging purpose is energy subtraction imaging, the target dose is determined such that the lung field, which is the region of interest, has a desired image quality in the radiation image acquired by the second radiation detector 6 on the side far from the radiation source 3. Accordingly, it is possible to ensure the image quality of the lung field in the radiation images acquired by both the first radiation detector 5 and the second radiation detector 6. Therefore, by the energy subtraction processing, it is possible to derive a bone part image in which the soft tissue is sufficiently removed in the lung field and a soft part image in which the bone part is sufficiently removed in the lung field.

The target dose in this case may be set as follows. That is, the chest phantoms having various thicknesses are imaged while changing the dose, and in the radiation image acquired by the second radiation detector 6, in a case in which the lung field has a desired image quality, the average reaching dose in the lung field region is set to the target dose and is stored in the storage 13. In a case in which the imaging is actually performed, the target dose stored in the storage 13 and corresponding to the imaging purpose may be read out from the storage 13 and used.

In a case in which the imaging purpose is energy subtraction imaging, there is a possibility that the dose is insufficient in the mediastinum and the subdiaphragmatic region in a case in which only the image quality of the lung field, which is the region of interest, is considered. In this case, even in a case in which the bone part image is derived by the energy subtraction processing, it is not possible to accurately separate the vertebrae and the soft tissue present in the mediastinum and the subdiaphragmatic region. Therefore, in a case in which the imaging part is the chest in a case in which the imaging purpose is energy subtraction imaging, in the radiation image acquired by the second radiation detector 6 on the side far from the radiation source 3, the mediastinum and the subdiaphragmatic region other than the lung field may be used as the region of interest, and the target dose may be determined such that the mediastinum and the subdiaphragmatic region have a desired image quality. Accordingly, it is possible to ensure the image quality of the entire image in the radiation images acquired by both the first radiation detector 5 and the second radiation detector 6.

Therefore, by the energy subtraction processing, it is possible to derive a bone part image in which the soft tissue is sufficiently removed for the vertebrae included in the mediastinum and the subdiaphragmatic region and a soft part image in which the bone part is sufficiently removed in the mediastinum and the subdiaphragmatic region.

Here, in the energy subtraction processing, whether to set the lung field as the region of interest or to set the mediastinum and the subdiaphragmatic region as the region of interest may be set based on the input from the input device 15 by the operator.

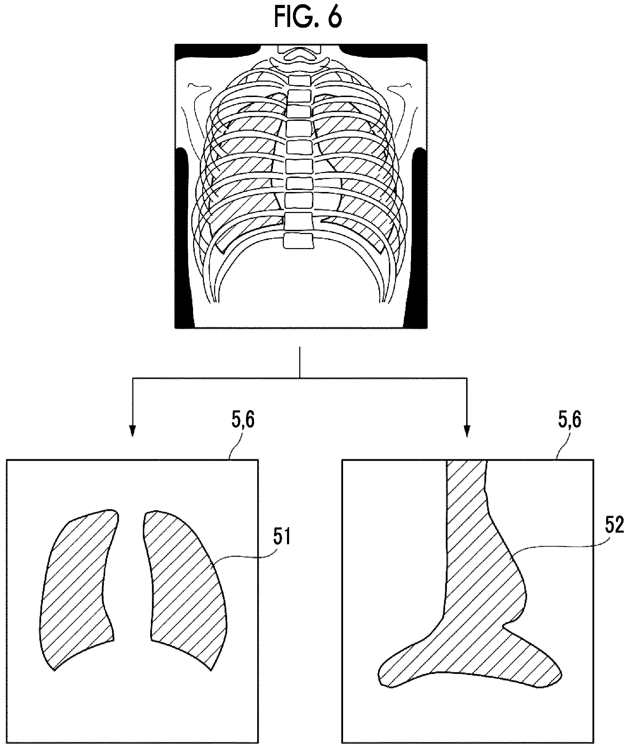

The region of interest of the subject H may be specified according to the imaging purpose, and the dose control may be performed according to the dose detected by the dose detection pixel 40B at a position corresponding to the region of interest in the radiation detector used for the dose control. For example, in a case in which the region of interest is a lung field, as shown in Fig. 6, the dose control is performed according to the dose detected by the dose detection pixel 40B at a position within a region 51 corresponding to the lung field in the radiation image in the radiation detectors 5 and 6.

On the other hand, in a case in which the region of interest is the mediastinum and the subdiaphragmatic region, as shown in Fig. 6, the dose control is performed according to the dose detected by the dose detection pixel 40B at a position within a region 52 corresponding to the mediastinum and the subdiaphragmatic region in the radiation image in the radiation detectors 5 and 6.

In a case in which the imaging purpose is simple imaging, the dose controller 21 derives a cumulative histogram of the dose data for each dose detection pixel 40B and specifies a subject region based on the cumulative histogram. Then, in a case in which the dose detection pixel 40B corresponding to the lung field is used, the dose controller 21 may derive a cumulative histogram of the dose data for each dose detection pixel 40B in the specified subject region and perform the dose control using the dose data acquired by the dose detection pixel 40B on the high dose side, which corresponds to 80% to 90% in the cumulative histogram.

On the other hand, in a case in which the imaging purpose is energy subtraction imaging, in a case in which the dose detection pixel 40B corresponding to the mediastinum and the subdiaphragmatic region is used, a cumulative histogram of the dose data may be derived for each dose detection pixel 40B in the specified subject region, and the dose control may be performed using the dose data acquired by the dose detection pixel 40B on the low dose side, which corresponds to 20% to 40% in the cumulative histogram.

In addition, the dose detection pixel 40B corresponding to the lung field or the mediastinum and the subdiaphragmatic region may be specified in advance, and the dose control may be performed using the dose detection pixel 40B specified according to the region of interest.

In a case in which the imaging part is the limbs or the like, the first radiation detector 5 may be determined as the radiation detector for dose control in a case in which the imaging purpose is simple imaging, and the second radiation detector 6 may be determined as the radiation detector for dose control in a case in which the imaging purpose is energy subtraction imaging. In this case, the region of interest is often a bone. Therefore, the subject region may be specified based on the cumulative histogram of the dose data, the cumulative histogram of the dose data may be derived for each dose detection pixel 40B in the specified subject region, and the dose control may be performed using the dose data acquired by the dose detection pixel 40B on the low dose side, which corresponds to 20% to 40% in the cumulative histogram.

The device controller 22 controls operations of the first and second radiation detectors 5 and 6 in response to an input operation from the operator via the input device 15. Specifically, the device controller 22 performs various controls such as turning on and off the power of the radiation detectors 5 and 6 and mode switching to a standby mode or an imaging mode. In addition, the device controller 22 preferably has a function of performing various types of image processing such as offset correction, sensitivity correction, and defect correction on the radiation images acquired by the first and second radiation detectors 5 and 6.

In a case in which the imaging purpose is energy subtraction imaging, the subtraction unit 23 derives a bone part image Gb in which only the bone part of the subject H included in the first radiation image G1 and the second radiation image G2 is extracted and a soft part image Gs in which only the soft part is extracted by performing weighting subtraction between corresponding pixels of the first radiation image G1 acquired by the first radiation detector 5 and the second radiation image G2 acquired by the second radiation detector 6, as shown in the following Equations (1) and (2). Note that $\alpha 1$ and $\alpha 2$ in Equation (1) and Equation (2) are weighting coefficients and are derived based on an attenuation coefficient according to the radiation energy of the soft part and the bone part of the subject H.

$$Gb(x,y) = G1(x,y) - \alpha 1 \times G2(x,y) \quad (1)$$

$$Gs(x,y) = G2(x,y) - \alpha 2 \times G1(x,y) \quad (2)$$

The display controller 24 displays the acquired radiation image on the display 14. That is, in a case in which the imaging purpose is simple imaging, the display controller 24 displays the radiation image acquired by the first radiation detector 5 on the display 14. In a case in which the imaging purpose is energy subtraction imaging, the display controller 24 displays the bone part image Gb and the soft part image Gs on the display 14.

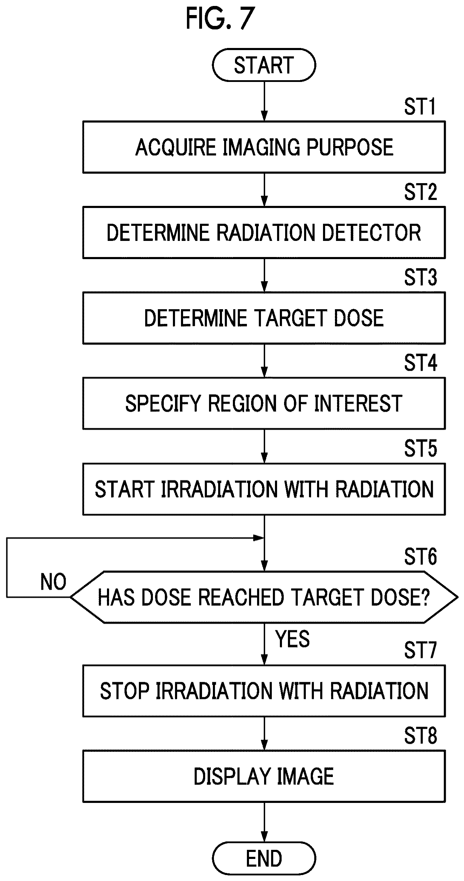

Next, processing performed in the present embodiment will be described. Fig. 7 is a flowchart showing the processing performed in the present embodiment. First, the dose controller 21 acquires the imaging purpose input by the operator through the input device 15 (Step ST1), and specifies at least one radiation detector used for dose control (Step ST2). In addition, the dose controller 21 determines a target dose to be irradiated onto the subject H according to the imaging purpose (Step ST3), and specifies a region of interest in the subject H according to the imaging purpose (Step ST4).

Then, in a case in which the irradiation switch 19 is operated, the dose controller 21 drives the radiation source 3 to start the irradiation with the radiation (Step ST5). Then, the dose controller 21 determines whether or not the dose during the imaging has reached the target dose based on dose data detected by the dose detection pixel 40B at a position corresponding to the region of interest in the radiation detector for dose control (Step ST6).

In a case in which the determination result in Step ST6 is "No", the irradiation with the radiation continues. In a case in which the determination result in Step ST6 is "Yes", the dose controller 21 stops the irradiation with the radiation by the radiation source 3 (Step ST7).

Then, the device controller 22 acquires a radiation image from the radiation detector according to the imaging purpose, and performs the energy subtraction processing as necessary. Then, the display controller 24 displays the acquired radiation image or bone part image and soft part image on the display 14 (image display: Step ST8), and ends the processing.

As described above, in the present embodiment, for the plurality of radiation detectors 5 and 6 included in the layer structure detector 4, at least one radiation detector to be used for dose control of radiation is determined according to the imaging purpose, and the dose control is performed according to the dose detected by the dose detection pixel 40B of the determined radiation detector. Therefore, in a case in which the radiography is performed using the layer structure detector 4, a high-quality simple radiation image, a bone part image, or a soft part image can be acquired. In the following, in a case in which it is not necessary to distinguish between the simple radiation image, the bone part image, and the soft part image, the simple radiation image, the bone part image, and the soft part image may be simply referred to as a radiation image.

In particular, in a case in which the imaging purpose is simple imaging, the first radiation detector 5 closest to the radiation source 3 among the plurality of radiation detectors 5 and 6 is determined as the radiation detector that performs the dose control of the radiation, and thus, a high-quality simple radiation image can be acquired.

In addition, in a case in which the imaging purpose is energy subtraction imaging, by determining the second radiation detector 6 other than the first radiation detector 5 closest to the radiation source 3 among the plurality of radiation detectors 5 and 6 as the radiation detector that performs the dose control of the radiation, it is possible to irradiate the second radiation detector 6 on a side away from the radiation source 3 with a sufficient dose of radiation. Therefore, the bone part image and the soft part image with high image quality can be acquired.

In addition, a higher quality radiation image can be acquired by determining a target dose according to the imaging purpose and performing the dose control such that the subject H is irradiated with the radiation of the target dose.

In addition, it is possible to acquire a radiation image with high image quality for the region of interest by specifying the region of interest in the subject H according to the imaging purpose and performing the dose control according to the dose detected by the dose detection pixel 40B corresponding to the region of interest.

In the above-described embodiment, in a case in which the imaging purpose is energy subtraction imaging, the second radiation detector 6 on the side far from the radiation source 3 is determined as the radiation detector to be used for the radiation source control, but the present disclosure is not limited thereto. Both the first radiation detector 5 and the second radiation detector 6 may be determined as a radiation detector for the radiation source control. In the following, this will be described as another embodiment.

Here, since the first radiation detector 5 on the side close to the radiation source 3 has a higher dose than the second radiation detector 6, a pixel value on the high density side may be saturated in the radiation image acquired by the first radiation detector 5. On the other hand, the radiation image acquired by the second radiation detector 6 may have a deteriorated S/N due to the insufficient dose.

For this reason, in a case in which the imaging part is the chest, in the radiation image acquired by the second radiation detector 6, in a case in which the dose is controlled to improve the image quality of the mediastinum and the subdiaphragmatic region, the pixel value of the lung field of the radiation image acquired by the first radiation detector 5 may be saturated. Therefore, in the other embodiment, the dose controller 21 may determine both the first radiation detector 5 and the second radiation detector 6 as radiation detectors for dose control.

In the other embodiment, in a case in which the imaging part of the subject H is the chest and the region of interest is the lung field, the dose controller 21 performs the dose control according to the dose detected by the dose detection pixel 40B at a position in a region corresponding to the lung field in the first radiation detector 5. In addition, in the second radiation detector 6, the dose controller 21 performs the dose control according to the dose detected by the dose detection pixel 40B at a position in a region corresponding to the mediastinum and the subdiaphragmatic region.

Specifically, the dose controller 21 sets an upper limit dose, which is an upper limit at which the pixel value of the radiation image is not saturated, in advance, and controls the drive of the radiation source 3 such that the irradiation with the radiation is stopped in a case in which the dose detected by the dose detection pixel 40B corresponding to the lung field region in the first radiation detector 5 is the upper limit dose or the dose detected by the dose detection pixel 40B corresponding to the mediastinum and the subdiaphragmatic region in the second radiation detector 6 is the target dose.

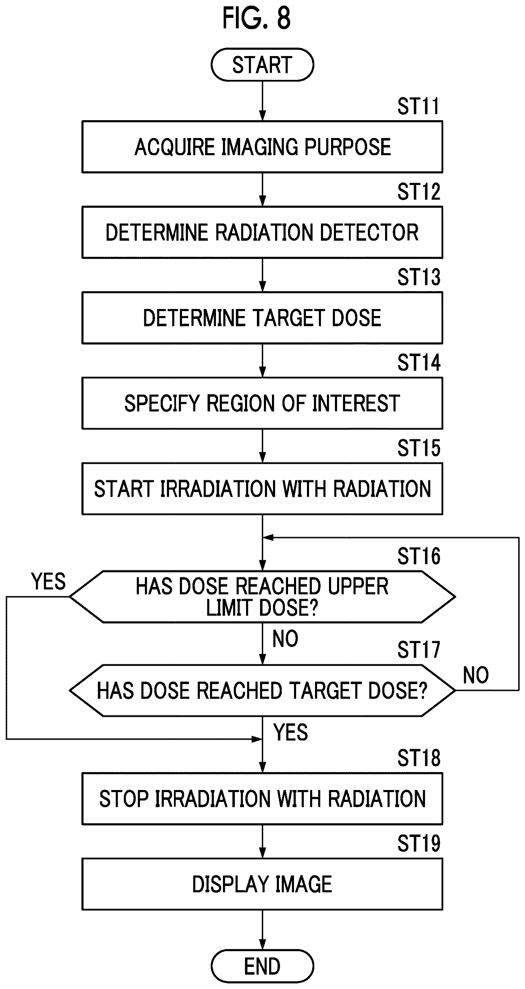

Next, processing performed in the other embodiment will be described. Fig. 8 is a flowchart showing the processing that is performed in the other embodiment. First, the dose controller 21 acquires the imaging purpose input by the operator through the input device 15 (Step ST11), and specifies at least one radiation detector used for dose control (Step ST12). In the other embodiment, since the imaging purpose is energy subtraction imaging, both the first radiation detector 5 and the second radiation detector 6 are specified as radiation detectors to be used for dose control. In addition, the dose controller 21 determines a target dose to be irradiated onto the subject H according to the imaging purpose (Step ST13), and specifies a region of interest in the subject H according to the imaging purpose (Step ST14). In the other embodiment, the region of interest is the lung field.

Then, in a case in which the irradiation switch 19 is operated, the dose controller 21 drives the radiation source 3 to start the irradiation with the radiation (Step ST15). Then, the dose controller 21 determines whether or not the dose irradiated onto the subject H has reached the upper limit dose based on dose data detected by the dose detection pixel 40B at a position corresponding to the region of interest in the first radiation detector 5 (Step ST16). In a case in which the determination result in Step ST16 is "No", it is determined whether or not the dose during the imaging has reached the target dose based on dose data detected by the dose detection pixel 40B at a position corresponding to the mediastinum and the subdiaphragmatic region in the second radiation detector 6 (Step ST17).

In a case in which the determination result in Step ST17 is "No", the processing returns to Step ST16, and the irradiation with the radiation continues. In a case in which the determination result in Step ST16 and Step ST17 is "Yes", the dose controller 21 stops the irradiation with the radiation by the radiation source 3 (Step ST18).

Then, the device controller 22 acquires a radiation image from the radiation detector according to the imaging purpose, and the subtraction unit 23 performs the energy subtraction processing. Then, the display controller 24 displays the bone part image and the soft part image derived by the energy subtraction processing on the display 14 (image display: Step ST19), and ends the processing.

As a result, according to the other embodiment, the dose can be controlled so that the region having a high density in the radiation image is not saturated as in a case in which the region of interest is the lung field. Therefore, a high-quality radiation image can be acquired.

The radiation in the embodiment described above is not particularly limited, and $\alpha$-rays or $\gamma$-rays can be used in addition to X-rays.

In addition, in the above-described embodiment, for example, the following various processors can be used as the hardware structure of processing units that execute various types of processing, such as the dose controller 21, the device controller 22, the subtraction unit 23, and the display controller 24. As described above, the various processors include, in addition to the CPU that is a general-purpose processor which executes software (program) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute a specific processing, such as an application specific integrated circuit (ASIC).

One processing unit may be configured by one of these various processors, or may be configured by combining two or more processors of the same type or different types (for example, by combining a plurality of FPGAs or combining the CPU and the FPGA). Further, a plurality of processing units may be composed of one processor.

As an example of configuring the plurality of processing units by one processor, first, as represented by a computer of a client, a server, and the like, there is an aspect in which one processor is configured by a combination of one or more CPUs and software and this processor functions as a plurality of processing units. Second, as represented by a system on chip (SoC) or the like, there is an aspect of using a processor that realizes the function of the entire system including the plurality of processing units by one integrated circuit (IC) chip.

In this way, various processing units are formed using one or more of the above-mentioned various processors as hardware structures.

Further, as the hardware structures of these various processors, more specifically, it is possible to use an electrical circuit (circuitry) in which circuit elements, such as semiconductor elements, are combined.

Hereinafter, description regarding supplementary notes of the present disclosure will be made.

(Supplementary Note 1)

A radiography control device that controls radiography of an imaging target by irradiating the imaging target with radiation emitted from a radiation source, the radiography control device comprising: a layer structure detector configured by stacking a plurality of radiation detectors each having a plurality of dose detection pixels for detecting a dose during the radiography; and at least one processor, in which the processor is configured to: acquire an imaging purpose; determine at least one radiation detector to be used for dose control of the radiation during the radiography among the plurality of radiation detectors according to the imaging purpose; and perform the dose control according to the dose detected by the dose detection pixel of the determined radiation detector.

(Supplementary Note 2)

The radiography control device according to Supplementary Note 1, in which the processor is configured to determine a radiation detector closest to the radiation source among the plurality of radiation detectors as the radiation detector for performing the dose control, in a case in which the imaging purpose is simple imaging.

(Supplementary Note 3)

The radiography control device according to Supplementary Note 1, in which the processor is configured to determine a radiation detector other than a radiation detector closest to the radiation source among the plurality of radiation detectors as the radiation detector for performing the dose control, in a case in which the imaging purpose is energy subtraction imaging.

(Supplementary Note 4)

The radiography control device according to Supplementary Note 1, in which the processor is configured to determine all of the plurality of radiation detectors as the radiation detector for performing the dose control, in a case in which the imaging purpose is energy subtraction imaging.

(Supplementary Note 5)

The radiography control device according to any one of Supplementary Notes 1 to 4, in which the processor is configured to: determine a target dose in a case in which the radiography is performed according to the imaging purpose; and stop driving of the radiation source in a case in which the dose detected by the dose detection pixel of the determined radiation detector reaches the target dose.

(Supplementary Note 6)

The radiography control device according to any one of Supplementary Notes 1 to 5, in which the processor is configured to: specify a region of interest in the imaging target according to the imaging purpose; and perform the dose control according to the dose detected by the dose detection pixel at least at a position corresponding to the region of interest in the radiation detector determined as the radiation detector that controls the dose of the radiation.

(Supplementary Note 7)

A radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method comprising: acquiring an imaging purpose; determining at least one radiation detector to be used for dose control of the radiation during the radiography among the plurality of radiation detectors according to the imaging purpose; and performing the dose control according to the dose detected by the dose detection pixel of the determined radiation detector.

(Supplementary Note 8)

A radiography control program causing a computer to execute a radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method including: a procedure of acquiring an imaging purpose; a procedure of determining at least one radiation detector to be used for dose control of the radiation during the radiography among the plurality of radiation detectors according to the imaging purpose; and a procedure of performing the dose control according to the dose detected by the dose detection pixel of the determined radiation detector.

Explanation of References

    1: imaging apparatus
    3: radiation source
    4: layer structure detector
    5, 6: radiation detector
    7: radiation energy conversion filter
    10: radiography control device
    11: CPU

12: radiography control processing program
13: storage
14: display
15: input device
16: memory
17: network I/F
18: high voltage generator
19: irradiation switch
20: bus
21: radiation source controller
22: device controller
23: subtraction unit
24: display controller
30: pixel region
31: gate driver
32: signal processing circuit
34: controller
35: communication I/F
40: pixel
40A: normal pixel
40B: detection pixel
41: photoelectric conversion unit
42: TFT
43: scanning line
44: signal line
51: region corresponding to lung field
52: region corresponding to mediastinum and subdiaphragmatic region
G1, G2: radiation image
Gb: bone part image
Gs: soft part image
H: subject

**Claims**

1. A radiography control device that controls radiography of an imaging target by irradiating the imaging target with radiation emitted from a radiation source, the radiography control device comprising:

   a layer structure detector configured by stacking a plurality of radiation detectors each having a plurality of dose detection pixels for detecting a dose during the radiography; and
   at least one processor,
   wherein the processor is configured to:

   acquire an imaging purpose;
   determine at least one radiation detector to be used for dose control of the radiation during the radiography among the plurality of radiation detectors according to the imaging purpose; and
   perform the dose control according to the dose detected by the dose detection pixel of the determined radiation detector.

2. The radiography control device according to claim 1, wherein the processor is configured to determine a radiation detector closest to the radiation source among the plurality of radiation detectors as the radiation detector for performing the dose control, in a case in which the imaging purpose is simple imaging.

3. The radiography control device according to claim 1, wherein the processor is configured to determine a radiation detector other than a radiation detector closest to the radiation source among the plurality of radiation detectors as the radiation detector for performing the dose control, in a case in which the imaging purpose is energy subtraction imaging.

4. The radiography control device according to claim 1, wherein the processor is configured to determine all of the plurality of radiation detectors as the radiation detector for performing the dose control, in a case in which the imaging purpose is energy subtraction imaging.

5. The radiography control device according to any one of claims 1 to 4,
   wherein the processor is configured to:

   determine a target dose in a case in which the radiography is performed according to the imaging purpose; and
   stop driving of the radiation source in a case in which the dose detected by the dose detection pixel of the determined radiation detector reaches the target dose.

6. The radiography control device according to claim 1, wherein the processor is configured to:

   specify a region of interest in the imaging target according to the imaging purpose; and
   perform the dose control according to the dose detected by the dose detection pixel at least at a position corresponding to the region of interest in the radiation detector determined as the radiation detector that controls the dose of the radiation.

7. A radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method comprising:

   acquiring an imaging purpose;

determining at least one radiation detector to be used for dose control of the radiation during the radiography among the plurality of radiation detectors according to the imaging purpose; and performing the dose control according to the dose detected by the dose detection pixel of the determined radiation detector.

8. A radiography control program causing a computer to execute a radiography control method in a radiography control device that controls radiography of an imaging target by irradiating the imaging target with radiation emitted from a radiation source, the radiography control device including a layer structure detector configured by stacking a plurality of radiation detectors each having a plurality of dose detection pixels for detecting a dose during the radiography, the radiography control method comprising:

a procedure of acquiring an imaging purpose; a procedure of determining at least one radiation detector to be used for dose control of the radiation during the radiography among the plurality of radiation detectors according to the imaging purpose; and a procedure of performing the dose control according to the dose detected by the dose detection pixel of the determined radiation detector.

# FIG. 1

1

H

5

6

4

3

7

10

RADIOGRAPHY CONTROL
DEVICE

# FIG. 2

# FIG. 3

5,6

40B

40A

# FIG. 4

10

5,6
RADIATION DETECTOR

11
CPU

16
MEMORY

17
NETWORK I/F

20

13
STORAGE

12
RADIOGRAPHY CONTROL PROGRAM

DISPLAY

14

INPUT DEVICE

15

18
HIGH VOLTAGE GENERATOR

IRRADIATION SWITCH
19

# FIG. 5

RADIOGRAPHY
CONTROL DEVICE — 10

DOSE CONTROLLER — 21

DEVICE CONTROLLER — 22

SUBTRACTION UNIT — 23

DISPLAY CONTROLLER — 24

# FIG. 6

5,6

5,6

51

52

# FIG. 7

```
          ╭─────────╮
          │  START  │
          ╰─────────╯
               │
               ▼                          ST1
  ┌─────────────────────────────────┐
  │     ACQUIRE IMAGING PURPOSE      │
  └─────────────────────────────────┘
               │
               ▼                          ST2
  ┌─────────────────────────────────┐
  │   DETERMINE RADIATION DETECTOR   │
  └─────────────────────────────────┘
               │
               ▼                          ST3
  ┌─────────────────────────────────┐
  │      DETERMINE TARGET DOSE       │
  └─────────────────────────────────┘
               │
               ▼                          ST4
  ┌─────────────────────────────────┐
  │     SPECIFY REGION OF INTEREST   │
  └─────────────────────────────────┘
               │
               ▼                          ST5
  ┌─────────────────────────────────┐
  │   START IRRADIATION WITH RADIATION │
  └─────────────────────────────────┘
               │
               ▼                          ST6
  NO  ◁─────────────────────────────────▷
      │   HAS DOSE REACHED TARGET DOSE?  │
      └─────────────────────────────────┘
               │ YES
               ▼                          ST7
  ┌─────────────────────────────────┐
  │   STOP IRRADIATION WITH RADIATION │
  └─────────────────────────────────┘
               │
               ▼                          ST8
  ┌─────────────────────────────────┐
  │          DISPLAY IMAGE           │
  └─────────────────────────────────┘
               │
               ▼
          ╭─────────╮
          │   END   │
          ╰─────────╯
```

# FIG. 8

```
START
```

ST11

ACQUIRE IMAGING PURPOSE

ST12

DETERMINE RADIATION DETECTOR

ST13

DETERMINE TARGET DOSE

ST14

SPECIFY REGION OF INTEREST

ST15

START IRRADIATION WITH RADIATION

ST16

YES ← HAS DOSE REACHED UPPER LIMIT DOSE?

NO

ST17

HAS DOSE REACHED TARGET DOSE? → NO

YES

ST18

STOP IRRADIATION WITH RADIATION

ST19

DISPLAY IMAGE

```
END
```

## INTERNATIONAL SEARCH REPORT

| International application No. |
| --- |
| **PCT/JP2023/042779** |

### A. CLASSIFICATION OF SUBJECT MATTER

*A61B 6/42*(2024.01)i; *A61B 6/00*(2024.01)i; *H05G 1/44*(2006.01)i
FI:    A61B6/42 500S; A61B6/00 520Z; A61B6/00 533; A61B6/00 550S; H05G1/44 A

According to International Patent Classification (IPC) or to both national classification and IPC

### B. FIELDS SEARCHED

Minimum documentation searched (classification system followed by classification symbols)

A61B6/42; A61B6/00; H05G1/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2020-193914 A (CANON KABUSHIKI KAISHA) 03 December 2020 (2020-12-03) entire text, all drawings | 1-8 |
| A | JP 2018-023769 A (FUJIFILM CORPORATION) 15 February 2018 (2018-02-15) entire text, all drawings | 1-8 |
| A | US 2008/0123802 A1 (CMT MEDICAL TECHNOLOGIES LTD.) 29 May 2008 (2008-05-29) entire text, all drawings | 1-8 |
| A | JP 2006-280576 A (FUJI PHOTO FILM CO., LTD.) 19 October 2006 (2006-10-19) entire text, all drawings | 1-8 |
| A | JP 59-145983 A (BARNES, Gary T.) 21 August 1984 (1984-08-21) entire text, all drawings | 1-8 |

☐ Further documents are listed in the continuation of Box C.    ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
| --- | --- |
| **01 February 2024** | **13 February 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2023/042779**

| Patent document cited in search report | | | Publication date (day/month/year) | Patent family member(s) | | | Publication date (day/month/year) |
|---|---|---|---|---|---|---|---|
| JP | 2020-193914 | A | 03 December 2020 | US 2022/0075085 A1 entire text, all drawings WO 2020/241062 A1 | | | |
| JP | 2018-023769 | A | 15 February 2018 | US 2018/0031714 A1 entire text, all drawings | | | |
| US | 2008/0123802 | A1 | 29 May 2008 | (Family: none) | | | |
| JP | 2006-280576 | A | 19 October 2006 | US 2006/0219926 A1 entire text, all drawings | | | |
| JP | 59-145983 | A | 21 August 1984 | US 4626688 A entire text, all drawings EP 115125 A1 | | | |

Form PCT/ISA/210 (patent family annex) (July 2022)

**REFERENCES CITED IN THE DESCRIPTION**

**Patent documents cited in the description**

- JP 2013233420 A